# EUROPEAN PATENT APPLICATION

(11) **EP 4 501 333 A1**
(43) Date of publication of application: **05.02.2025**
(21) Application number: 23781024.7
(22) Date of filing: 30.03.2023
(51) Int. Cl.: A61K 31/7068, A61K 8/60, A61K 31/7072, A61P 9/10, A61P 11/00, A61P 17/00, A61P 43/00, A61Q 19/00

(54) **FIBRILLIN PRODUCTION PROMOTER, COSMETIC COMPOSITION OR SKIN TOPICAL AGENT, ORALLY ADMINISTERED AGENT, AND ELASTIN FIBER PRODUCTION PROMOTER**

(30) Priority: 30.03.2022 JP 2022056621
(71) Applicant: YAMASA CORPORATION, Choshi-shi, Chiba 288-0056 (JP)
(72) Inventor: ISHIGE,Kazuya, Choshi-shi, Chiba 288-0056 (JP)
(74) Representative: Maiwald GmbH
(86) International application number: PCT/JP2023/013439
(87) International publication number: WO 2023/191016

(57) **Abstract**

A fibrillin production promoter contains at least one pyrimidine nucleotide or precursor thereof as an active ingredient.

## Description

### [Technical Field]

The present invention relates to a fibrillin production promoter, a cosmetic composition or a skin topical agent, an oral administering agent, and an elastin fiber production promoter.

### [Background Art]

In today's aging society, healthy longevity as well as improvement of QOL (Quality of Life) have become issues. Skin beauty in the anti-aging field is very important for improving the quality of life of elderly people, and so there is growing interest.

A "skin elasticity" is often cited as an element of ideal skin, and is an element with high demand for improvement in order to improve QOL. Recent studies have revealed that the "elasticity" has two elements: "visual elasticity" and "tactile elasticity" (Non-Patent Literature 1). In Non-Patent Literature 1, the relationship between the "visual elasticity" and the "tactile elasticity" was studied, and the "visual elasticity" was strongly correlated with a "visual moist feeling", while the "tactile elasticity" was not correlated with the "visual moist feeling". Non-Patent Literature 1 discloses that significant improvement of the "visual elasticity" was observed with an increase in a stratum corneum moisture content, while no significant change in the "tactile elasticity" was observed.

Elastic fibers (elastin fibers) are known to play an important role in the "tactile elasticity. The elastin fibers in the skin dermis form a continuous network that extends from the basement membrane to the subcutaneous tissue, thereby maintaining the elasticity of the skin. A change due to degeneration of elastin fibers is a major factor in the skin changes associated with aging, and is said to be a major cause of skin sagging and wrinkling. In natural aging, elastin fibers are known to decrease. In photoaging, on the other hand, fibers in the papillary layer of the dermis disappear, as in solar elastosis, but amounts of elastin fibers in the reticular layer increase and degenerate, resulting in deposition like amorphous substances. It is believed that these are the cause of photoaged skin that lacks elasticity and is hardened and wrinkled.

Elastic fibers are abundant in the lungs and arteries, besides the skin, and are responsible for the elasticity of these tissues. Age-related deterioration of elastic fibers is a direct cause of pulmonary emphysematous changes, arterial tunica media stiffness and the like (Non-Patent Literature 2).

As described above, deterioration or rupture of elastic fibers causes symptoms such as a loss of tactile elasticity of the skin, emphysematous changes, and arterial medial sclerosis, so that maintenance and regeneration of elastic fibers is an urgent issue to improve QOL. However, the turnover of elastic fibers is extremely slow, and aged tissues have substantially no ability to regenerate elastic fibers (Non-Patent Literature 2). If elastic fibers could be regenerated, it would not only improve the tactile elasticity of the skin, but also create new preventive and therapeutic methods, such as the prevention and treatment of the development of emphysema and atherosclerosis.

Microfibrils (fibrillin) and amorphous elastin are known to be the major components of elastic fibers. The elastic fibers are formed by the deposition of elastin onto the microfibrils. In other words, the production of elastic fibers would be promoted by promoting the production of microfibrils.

Citidylic acid and uridylic acid are a class of nucleotides and are widely found in living organisms and foods, and can be safe materials.

Patent Literature 1 discloses a beauty composition comprising uridine or a derivative thereof and acetylglucosamine or the like. Patent Literature 1 teaches that skin moisturizing properties are improved to increase a stratum corneum moisture content, and the skin elasticity is maintained.

In view of Non-Patent Document 1 which teaches that significant improvement of "visual elasticity" was observed with an increase in a stratum corneum water content, while no significant change in "tactile elasticity" was observed, it is understood that the "elasticity" of the skin in Patent Literature 1 is the so-called "visual elasticity". Patent Literature 1 does not disclose that pyrimidine nucleotides or their precursors promote the production of fibrillin or contribute to improvement of the "tactile elasticity" of the skin.

Patent Literature 2 discloses that a phosphate ester of uridine or a physiologically acceptable salt thereof promotes hyaluronic acid production in cells and contributes to moisturizing, and the like, and that it also promotes cell proliferation. Patent Literature 2 also discloses that it improves wrinkling and sagging of the skin due to the promotion of hyaluronic acid production and cell proliferation.

It is considered that the improvement of wrinkling and sugging of the skin disclosed in Patent Literature 2 is the improvement of the so-called "visual elasticity" of the skin due to moisturization of the skin by promoting hyaluronic acid production, and the appearance change resulting from quantitative changes due to cell proliferation. Patent Literature 2 does not disclose that pyrimidine nucleotides or their precursors promote the fibrillin production and contribute to the improvement of the "tactile elasticity" of the skin in any way.

Patent Literature 3 describes a composition for promoting fibrillin production, the composition comprising a purine-based nucleic acid-related substance and a pyrimidine-based nucleic acid-related substance. However, the invention substantially disclosed in Patent Literature 3 is the composition for promoting fibrillin production in combination with adenylic acid and uridylic acid, and there is no substantial disclosure for the use of uridylic acid alone and the use of cytidylic acid. Furthermore, there is no disclosure in Patent Literature 3 that pyrimidine nucleotides or their precursors promote fibrillin production or contribute to improvement of the "tactile elasticity" of the skin.

Therefore, it has not been evaluated whether nucleic acid-related substances such as cytidylic acid and uridylic acid have an effect of promoting fibrillin production.

### [Citation List]

### [Patent Literatures]

[PTL 1]
   Japanese Patent Application Publication No. 2014-88329 A
[PTL 2]
   WO 2019/021988 A1
[PTL 3]
   WO 2005/034902 A1

### [Non-Patent Literatures]

[NON-PTL1]
   Kazunori Kobayashi, et.al., "Study for Visual Elasticity", Journal of the Japanese Society of Cosmetic Technologists, Vol. 45, No. 3, 2011, p. 212-217
[NON-PTL1]
   Tomoyuki Nakamura, "Mechanism for Creating Stretchability in Living Organisms", Journal of the Japanese Society of Cosmetic Science, Vol. 43, No. 1, pp. 14-17 (2019)

### [Summary of Invention]

### [Technical Problem]

An object of the present invention is to provide a novel and highly safe fibrillin production promoter, a cosmetic composition or a skin topical agent, and an oral administering agent, which have an effect of promoting fibrillin production.

Another object of the present invention is to provide a novel and highly safe elastin fiber production promoter that has an effect of promoting elastin fiber production.

### [Solution to Problem]

As a result of intensive studies to achieve the above object, surprisingly, the present inventors have firstly found that pyrimidine nucleotides or precursors thereof, which were not known to have the effect of promoting fibrillin production, have an attribute of promoting fibrillin production and elastin fiber production, and they have completed the present invention.

Thus, the present invention is a fibrillin production promoter comprising at least one pyrimidine nucleotide or precursor thereof as an active ingredient.

The present invention is also a cosmetic composition or a skin topical agent for promoting fibrillin production, wherein the cosmetic composition or the skin topical agent comprises at least one pyrimidine nucleotide or precursor thereof as an active ingredient.

Further, the present invention is an oral administering agent for promoting fibrillin production, wherein the preparation comprises at least one pyrimidine nucleotide or precursor thereof as an active ingredient.

Furthermore, the present invention is an elastin fiber production promoter comprising at least one pyrimidine nucleotide or precursor thereof as an active ingredient.

### [Advantageous Effects of Invention]

The fibrillin production promoter, the cosmetic composition or skin topical agent, and the oral administering agent according to the present invention have an effect of promoting fibrillin production, and are novel and highly safe.

Also, the elastin fiber production promoter according to the present invention has an effect of promoting elastin fiber production, and is novel and highly safe.

### [Brief Description of Drawings]

[FIG. 1]

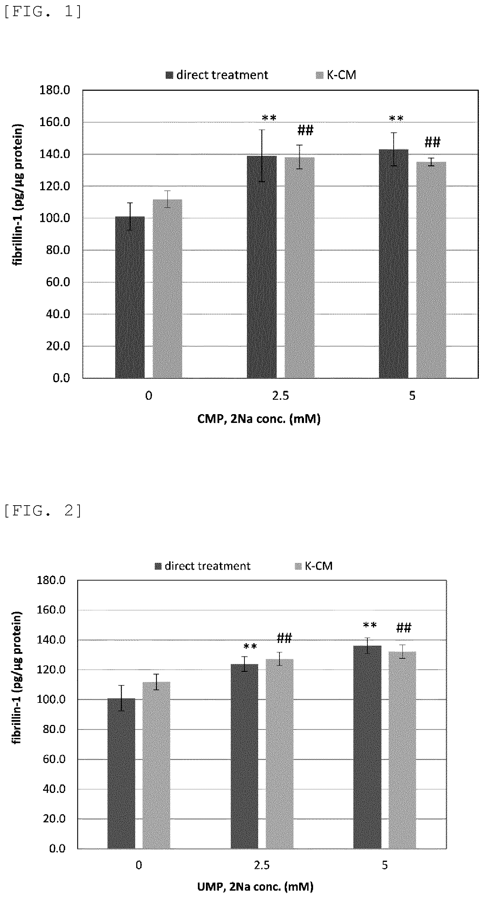

   FIG. 1 shows quantified results of an amount of fibrillin-1 (ng/µg protein) quantified in Example 1 after a CMP, 2Na treatment. In the figure, the "direct treatment" shown in the dark gray color means measured results of samples in which the samples were directly treated with normal human fibroblasts. The "K-CM" shown in the grayish white color means measured results of samples in which a culture supernatant of normal human epidermal cells was treated with normal human fibroblasts. Each value on the horizontal axis means the CMP, 2Na concentration (mM) in each sample. The symbol ** means that the p value is smaller than 0.01 when compared with the result of the direct treatment at a CMP, 2Na concentration of 0 (mM) in Student's t-test. The symbol ## means that the p value is smaller than 0.01 when compared with the result of K-CM at a CMP, 2Na concentration of 0 (mM) in Student's t-test.
[FIG. 2]
   FIG. 2 shows quantified results of an amount of fibrillin-1 (ng/µg protein) quantified in Example 1 when treated with UMP, 2Na. In the figure, the "direct treatment" shown in the dark gray color means measured results of samples in which the samples were directly treated with normal human fibroblasts. The "K-CM" shown in the grayish white color means measured results of samples in which normal human fibroblasts were treated with the culture supernatant of normal human epidermal cells. Each value on the horizontal axis means the concentration (mM) of UMP, 2Na in each sample. The symbol ** means that the p value is smaller than 0.01 when compared with the result of the direct treatment at a UMP, 2Na concentration of 0 (mM) in Student's t-test. The symbol ## means that the p value is smaller than 0.01 when compared with the results of K-CM at a UMP, 2Na concentration of 0 (mM) in Student's t-test.
[FIG. 3]

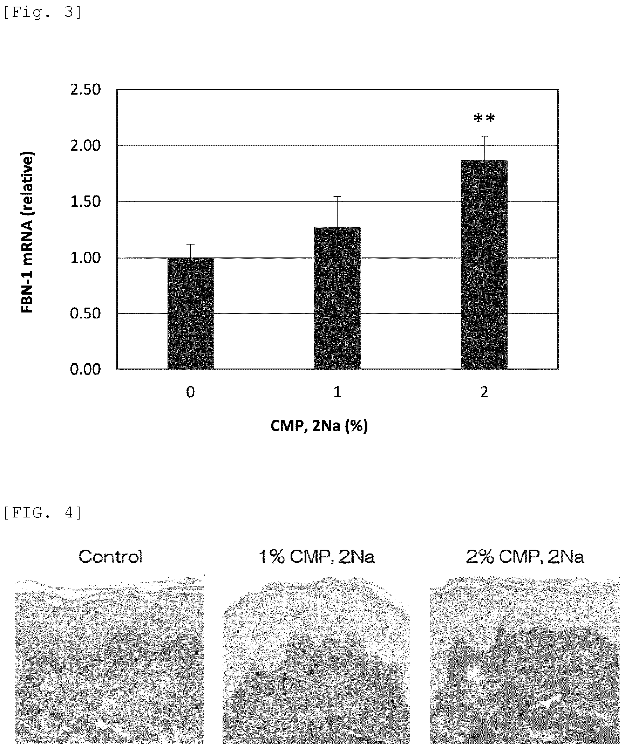

   FIG. 3 shows results of analysis of FBN-1 mRNA expression quantified in Example 2. Each value on the horizontal axis means a CMP, 2Na concentration (w/v%) in each sample. Each value on the vertical axis means a relative expression level, when the expression level of FBN-1 at a CMP, 2Na concentration of 0 (w/v%) is 1.00. The symbol ** means that the p value is smaller than 0.01 when compared with a result of a CMP, 2Na concentration of 0 (w/v%) in Student's t-test.
[FIG. 4]
   FIG. 4 shows photographs of results of elastica-van Gieson staining described in Example 3. The black areas in the dermis in the photographs represent elastin fibers. In the figure, the "Control" shows a result of a sample treated with CMP, 2Na at a concentration of 0 (w/v%). The "1% CMP, 2Na" shows a result of a sample treated with CMP, 2Na at a concentration of 1.0 (w/v%), and the "2% CMP, 2Na" shows a result of a sample treated with CMP, 2Na at a concentration of 2.0 (w/v%).

### [Description of Embodiments]

The present invention relates to a fibrillin production promoter, a cosmetic composition or skin topical agent, and an oral administering agent, which are for promoting fibrillin production and contain at least one pyrimidine nucleotide or precursor thereof as an active ingredient. The present invention also relates to an elastin fiber production promoter comprising at least one pyrimidine nucleotide or precursor thereof as an active ingredient.

As used herein, the "fibrillin production promoter" is a concept including a form of a composition that contains at least one pyrimidine nucleotide or precursor thereof as an active ingredient and further contains other ingredients. Similarly, as used herein, the "skin topical agent" and "elastin fiber production promoter" are concepts including a form of a composition that contains at least one pyrimidine nucleotide or precursor thereof as an active ingredient and further contains other ingredients.

As used herein, the pyrimidine nucleotide means cytidylic acid and/or uridylic acid.

The cytidylic acid (cytidine monophosphate, cytidine 5'-phosphate, CMP) is a compound represented by CAS Registry Number 63-37-6. When cytidylic acid is mentioned herein, salts of cytidylic acid are also included.

When a mass of cytidylic acid is described herein, it represents a mass when converted to cytidylic acid disodium salt (CMP,2Na). When a concentration (%) of a cytidylic acid solution is mentioned herein, it is a mass volume percent concentration (w/v%) unless otherwise specified, and a mass converted to CMP,2Na is used as the mass of cytidylic acid. If a salt other than the disodium salt is selected, or if it is a free acid that does not form a salt, it is a mass when converted to CMP,2Na, based on the amount of substance of the cytidylic acid.

Uridylic acid (uridine monophosphate, uridine 5'-phosphate, UMP) is a compound represented by CAS registration number 58-97-9. When the term "uridylic acid" is used herein, it is concept also including salts of uridylic acid.

When a mass of uridylic acid is described herein, it represents a mass when converted to uridylic acid disodium salt (UMP,2Na). When a concentration (%) of uridylic acid is mentioned herein, it is a mass volume percent concentration (w/v%) unless otherwise specified, and a mass converted to UMP,2Na is used as the mass of uridylic acid. If a salt other than the disodium salt is selected, or if it is a free acid that does not form a salt, it is a mass when converted to UMP,2Na, based on the amount of substance of the uridylic acid.

As used herein, the pyrimidine nucleotide precursor means a compound that can be metabolized to the pyrimidine nucleotide, i.e., cytidylic acid and/or uridylic acid. Whether a compound is included in the pyrimidine nucleotide precursor is determined by the presence or absence of knowledge that the compound is converted to the pyrimidine nucleotide. Specifically, cytidine diphosphate, cytidine triphosphate, uridine diphosphate, and uridine triphosphate, which are known to be degraded to cytidylic acid and/or uridylic acid by the action of ectonucleotidases and like (Isao Matsuoka, "Ectonucleotidase in Nervous System", Japanese Journal of Clinical Chemistry 33: 11-18, 2004), and cytidine, cytosine, uridine, and uracil, which are known to be phosphorylated to cytidylic acid and/or uridylic acid by the action of kinases (A Orengo, "Regulation of enzymic activity by metabolites. I. Uridine-cytidine kinase of Novikoff ascites rat tumor", J Biol Chem. 1969 Apr 25; 244(8): 2204-9.) are exemplified as the pyrimidine nucleotide precursors as used herein.

As used herein, the effect of promoting fibrillin production according to the present invention is evaluated by quantifying an amount of fibrillin-1. A specific method for the quantification will be described in Examples below.

As used herein, the tactile elasticity of the skin refers to the sensation when touching the skin, which is recognized from the elasticity when touched, the moist feeling when touched, and the elasticity when touched. For the meanings indicated by the terms "visual elasticity of the skin" and the "tactile elasticity of the skin" as used herein, reference may be made to Non-Patent Literature 1.

Examples of the pyrimidine nucleotide or precursor thereof in the present invention include, as described above, cytidine, cytosine, cytidylic acid, cytidine diphosphate, cytidine triphosphate, uridine, uracil, uridylic acid, uridyl diphosphate, and uridyl triphosphate. Among them, cytidylic acid and uridylic acid are preferable.

The concept of cytidylic acid as used herein includes salts as described above. The salts of cytidylic acid include alkali metal salts such as sodium salts and potassium salts; alkaline earth metal salts such as calcium salts, magnesium salts and barium salts; basic amino acid salts such as arginine and lysine; ammonium salts such as ammonium salts and tricyclohexylammonium salts; and various alkanolamine salts such as monoethanolamine salts, diethanolamine salts, triethanolamine salts, monoisopropanolamine salts, diisopropanolamine salts and triisopropanolamine salts. Among them, the alkali metal salts such as sodium salts are preferred. Specific examples of the alkali metal salts include monosodium cytidylate and disodium cytidylate. Disodium cytidylate is preferred in terms of handling.

The concept of uridylic acid as used herein includes salts as described above. The salts of uridylic acid include alkali metal salts such as sodium salts and potassium salts; alkaline earth metal salts such as calcium salts, magnesium salts and barium salts; basic amino acid salts such as arginine and lysine; ammonium salts such as ammonium salts and tricyclohexylammonium salts; and various alkanolamine salts such as monoethanolamine salts, diethanolamine salts, triethanolamine salts, monoisopropanolamine salts, diisopropanolamine salts and triisopropanolamine salts. Among them, the alkali metal salts such as sodium salts are preferred. Specific examples of the alkali metal salts include monosodium uridylate and disodium uridylate. Disodium uridylate is preferred in terms of handling.

In the fibrillin production promoter, the cosmetic composition or skin topical agent, and the oral administering agent according to the present invention, the pyrimidine nucleotide or the precursor thereof as the active ingredient may be used alone, or two or more types of pyrimidine nucleotides or precursors thereof may be simultaneously used, or the pyrimidine nucleotide or the precursor thereof may be used in combination with other active ingredients.

There is no particular limitation on the origins of the pyrimidine nucleotides or precursors thereof, and those derived from natural products such as yeast, bacteria, fish and shellfish, animals, and plants are suitable.

The fibrillin production promoter according to the present invention can be used for oral administrating agents. Specifically, it can be practically used for food and drink products, supplements, prepared milk powder, enteral nutritional supplements, healthy food and drink products (including food for specified health uses and foods with function claims), additives for animal feed, and pharmaceutical products for human or animals other than human.

When the fibrillin production promoter according to the present invention is provided as the food and drink product, health food and drink product or prepared milk powder, it can be made into a food or drink product having an effect of promoting fibrillin production by adding the above active ingredients to the known food and drink product as appropriate. The food and drink products of interest include milk and dairy products, seasonings, beverages, confectioneries, breads, noodles, oils and fats, processed meat products, processed marine products, processed agricultural products, frozen foods, and instant foods.

The novel food and drink products that have the effect of promoting fibrillin production can also be produced by mixing the pyrimidine nucleotides or precursors thereof with materials for the food and drink products. The shape of the food and drink products of interest can be selected from various forms, such as tablets, granules, capsules, powders, solutions, syrups, emulsions, and pastes. In addition to the active ingredient according to the present invention, various excipients and seasoning ingredients that can be used as foods may be added as needed in the production of those food products.

The food and drink product as described above may be provided and sold as a food and drink product labeled with the health application for promoting the fibrillin production. The act of "labeling" includes all acts to make the above application known to users, and all expressions that may evoke or analogize the above application fall under the act of "labeling" in this invention, regardless of the purpose of the labeling, the content of the labeling, and the object or medium to be labeled.

It is preferable that the above "labeling" be made by means of expressions that enable users to directly recognize the above application. Specific examples include the act of assigning, delivering, displaying for the purpose of assignment or delivery, or importing of goods or packages of the goods in relation to the food and drink products, which describe the above application, or the act of displaying or distributing advertisement materials, price lists or transaction documents in relation to the goods, which describes the above application, and the act of providing information about these contents, which describes the above application, through electromagnetic means (e.g., through the internet).

It is preferable that the contents of the labeling are those approved by the government or the like (e.g., labeling that has been approved based on various systems established by the government and is performed in a manner based on such approval). It is also preferable to attach such labeling to packages, containers, catalogs, pamphlets, POP, and other promotional materials at the places of sales, and other documents.

When the fibrillin production promoter according to the present invention is practically provided as a pharmaceutical, supplement, enteral nutritional product, and the like for oral administration, the above active ingredient can be formulated alone or in combination with formulation aids or the like.

The formulation may be in the form of a tablet, granule, capsule, granule, powder, solution, syrup, emulsion, and the like.

In the formulation described above, in addition to the active ingredient according to the present invention, any formulation auxiliaries such as excipients, binders, disintegrants, lubricants, flavoring agents, solubilizing aids, suspending agents, coating agents, and the like can be appropriately combined according to each dosage form.

The amount of the active ingredient in the agent according to the present invention may be appropriately selected from the range of 0.1 to 30% (W/W) depending on the purpose of use (prevention, health care, symptom relief, etc.), the age of the subject, the method of administration or ingestion, the dosage form, and the like.

Although an amount of the oral administering agent according to the present invention to be administered or ingested varies depending on the age, body weight, and severity of symptoms of the subject, the method of administration or ingestion, and the like, it may be selected from the range of about 1 mg to about 800 g per day. The amount of the oral administrating agent according to the present invention to be administered or ingested may preferably be 1 to 5000 mg, and more preferably 1 to 1000 mg, per dosage.

The fibrillin production promoter according to the present invention can also be put to practical use as a cosmetic composition or a skin topical agent.

The content of the pyrimidine nucleotide or the precursor thereof in the cosmetic composition or the skin topical agent according to the present invention is preferably 0.05 (w/v%) or more in terms of promoting fibrillin production. In particular, the effect of promoting fibrillin production is exerted in a concentration-dependent manner, and thus, the content is preferably 0.075 (w/v%) or more. Also, in terms of significantly promoting fibrillin production, the content is preferably 0.092 (w/v%) or more, and more preferably 0.25 (w/v%), and still more preferably 1.0 (w/v%) or more. Also, from the viewpoint of ease of handling when it is used as various agents or compositions, the content of pyrimidine nucleotide or the precursor thereof is preferably 10 (w/v%) or less.

The cosmetic composition or the skin topical agent according to the present invention may be prepared in various forms by combining pharmaceutically or cosmetically acceptable bases or carriers in addition to the above ingredients. Conventionally known bases and carriers can be used as the pharmaceutically or cosmetically acceptable bases and carriers. Also, for the cosmetic composition or the skin topical agent according to the present invention, it may optionally contain various known ingredients that will be formulated in external compositions applied to the skin or mucous membranes, such as cosmetics, external pharmaceuticals and quasi-drugs. Such ingredients include, for example, surfactants, coloring matters (dyes, pigments), fragrances, preservatives, bactericides (antibacterial agents), thickening agents, antioxidants, sequestering agents, cooling agents, deodorants, moisturizers, UV absorbers, UV scattering agents, vitamins, plant extracts, skin astringents, anti-inflammatory agents (antiphlogistic agents), whitening agents, cell activators, vasodilator agents, blood circulation promoters, skin function enhancers, and the like.

Among the above ingredients, specific examples of the surfactants include anionic surfactants such as higher fatty acid soaps, alkyl sulfate ester salts, polyoxyethylene alkyl ether sulfates, alkyl ether phosphate ester salts, N-acylamino acid salts, and acyl N-methyl taurine salts; cationic surfactants such as alkyltrimethylammonium chloride, and dialkyldimethylammonium chloride; amphoteric surfactants such as alkyl dimethyl amino acetic acid betaine, alkyl amido dimethyl amino acetic acid betaine, and 2-alkyl-N-carboxy-N-hydroxyimidazolinium betaine; nonionic surfactants such as polyoxyethylene type, polyhydric alcohol ester type, and ethylene oxide-propylene oxide block copolymers. Further, surfactants belonging to polymer surfactants or natural surfactants can also be used without limitation.

Specific examples of preservatives include ethyl paraoxybenzoate, salicylic acid, and sorbic acid. Specific examples of the thickening agent include xanthan gum, sodium carboxymethylcellulose, and carboxyvinyl polymers. Specific examples of the sequestering agent include sodium salt of ethylenediaminetetraacetic acid, phosphoric acid, and citric acid.

Specific examples of the moisturizers include polyethylene glycol, propylene glycol, glycerin, 1,3-butylene glycol, xylitol, sorbitol, maltitol, chondroitin sulfate, hyaluronic acid, mucoitinsulfuric acid, charonic acid, atelocollagen, sodium lactate, bile salts, dl-pyrrolidone carboxylate, short-chain soluble collagen, diglycerin (EO) PO adducts, chestnut rose extracts, yarrow extracts, and melilot extracts.

Specific examples of the vitamins include classes of vitamin A such as vitamin A oil, retinol, retinol acetate, and retinol palmitate; classes of vitamin B2 such as riboflavin, riboflavin butyrate, and flavin adenine nucleotides; classes of vitamin B6 such as pyridoxine hydrochloride, and pyridoxine dioctanoate; classes of vitamin C such as L-ascorbic acid, L-ascorbyl dipalmitate, L-ascorbic acid-2-sodium sulfate, and dl-α-tocopherol-L-ascorbic acid phosphate diester dipotassium; pantothenic acids such as calcium pantothenate, D-pantothenyl alcohol, pantothenyl ethyl ether, and acetyl pantothenyl ethyl ether: classes of vitamin D such as ergocalciferol and cholecalciferol; nicotinic acids such as nicotinic acid, benzyl nicotinate, and nicotinamide; classes of vitamin E such as dl-α-tocopherol, dl-α-tocopherol acetate, dl-α-tocopherol nicotinate, and dl-α-tocopherol succinate; and vitamin P, biotin, and the like.

The cosmetic composition or skin topical agent according to the present invention is used in a form to be applied to the skin (for example, by coating or spraying). Specifically, the cosmetic composition or skin topical agent according to the present invention is used as a cosmetic, an external pharmaceutical, or an external quasi-drug. Among these, the cosmetic is preferred in terms of promoting fibrillin production on a daily basis. Such a cosmetic or external preparation includes various hair cosmetics such as hair tonics or hair growth agents, or shampoos, rinses, and hair lotions (including tonics and liquids) that are effective for hair tonic or hair growth.

The form of the cosmetic composition or the skin topical agent according to the present invention is not particularly limited as long as it can be applied to the skin or mucous membranes, and examples include pastes, mousses, gels, liquids, emulsions, suspensions, creams, ointments, sheets, aerosols, sprays, and liniments. In particular, when it is used as a cosmetic, examples include lotions; emulsions such as emollient emulsions, milky lotions, nourishing emulsions, and cleansing emulsions; creams such as emollient creams, massage creams, cleansing creams, and makeup creams. Further, in particular, when it is used as a hair care product such as hair growth agents and hair restoration agents, examples include tonics, hair creams, hair lotions, aerosols (sprays), mousses, shampoos, conditioners, and liquids.

The cosmetic composition or the skin topical agent according to the present invention can be used by directly applying, spraying, or pasting it onto the skin or mucous membranes as a cosmetic, an external pharmaceutical, or an external quasi-drug. The rate of use can be appropriately selected depending on the user (age, sex, purpose, severity of symptoms in the affected area, and the like, for human) and is not particularly limited. For example, an effective amount to promote fibrillin production may be transdermally administered to the skin from once per day to five times per day.

An amount of application when applying the cosmetic composition or the skin topical agent according to the present invention is preferably 1 mg or more per dose as converted to the pyrimidine nucleotide or its precursor, more preferably 1 to 1000 mg, and even more preferably 5 to 800 mg, and still more preferably 10 to 500 mg.

As is clear from the contents previously described, the present invention is based on the findings that the pyrimidine nucleotide or the precursor thereof has a novel property of promoting fibrillin production, and that the property allows the pyridine nucleotide itself to be suitable for a novel application of a fibrillin production promoter.

One aspect of the present invention is an elastin fiber production promoter that promotes the production of elastin fibers. The elastin fiber production promoter according to the present invention can promote the production of elastin fibers by promoting the production of fibrillin.

The applied amount and administered amount of the elastin fiber production promoter can be the same as the applied amount when applying the above cosmetic composition or topical skin agent, and the applied amount of the oral administering agent.

The elastin fiber production promoter according to the present invention can achieve one or more effects selected from improvement of tactile elasticity of the skin, treatment of emphysema, prevention of the progression of emphysema, treatment of arteriosclerosis, and prevention of the progression of arteriosclerosis by promoting the fibrillin production.

Therefore, the present invention is illustrated below:
[1] A fibrillin production promoter comprising at least one pyrimidine nucleotide or precursor thereof as an active ingredient.
[2] The fibrillin production promoter according to [1], wherein a concentration of the pyrimidine nucleotide or precursor thereof is 0.05 to 10.0 (w/v%).
[3] The fibrillin production promoter according to [1] or [2], wherein the fibrillin production promoter has one or more effects selected from improvement of tactile elasticity of a skin, treatment of emphysema, prevention of progression of emphysema, treatment of arteriosclerosis, and prevention of progression of arteriosclerosis.
[4] A cosmetic composition or skin topical agent for promoting fibrillin production, the cosmetic composition or skin topical agent comprising at least one pyrimidine nucleotide or precursor thereof as an active ingredient.
[5] The cosmetic composition or skin topical agent according to [4], wherein a concentration of the pyrimidine nucleotide or precursor thereof is 0.05 to 10.0 (w/v%).
[6] The cosmetic composition or skin topical agent according to [4] or [5], wherein the cosmetic composition or skin topical agent has one or more effects selected from improvement of tactile elasticity of a skin, treatment of emphysema, prevention of progression of emphysema, treatment of arteriosclerosis, and prevention of progression of arteriosclerosis.
[7] An oral administering agent for promoting fibrillin production, the oral administering agent comprising at least one pyrimidine nucleotide or precursor thereof as an active ingredient.
[8] The oral administering agent according to [7], wherein a concentration of the pyrimidine nucleotide or precursor thereof is 0.05 to 10.0 (w/v%).
[9] The oral administering agent according to [7] or [8], wherein the oral administering agent has one or more effects selected from improvement of tactile elasticity of a skin, treatment of emphysema, prevention of progression of emphysema, treatment of arteriosclerosis, and prevention of progression of arteriosclerosis.
[10] An elastin fiber production promoter comprising at least one pyrimidine nucleotide or precursor thereof as an active ingredient.
[11] The elastin fiber production promoter according to [10], wherein a concentration of the pyrimidine nucleotide or precursor thereof is 0.05 to 10.0 (w/v%).
[12] The elastin fiber production promoter according to [10] or [11], wherein the elastin fiber production promoter has one or more effects selected from improvement of tactile elasticity of a skin, treatment of emphysema, prevention of progression of emphysema, treatment of arteriosclerosis, and prevention of progression of arteriosclerosis.
[13] A method for promoting fibrillin production in a skin, the method comprising applying the cosmetic composition or skin topical agent according to [4] or [5] to the skin.
[14] The method according to [13], wherein an amount of the cosmetic composition or skin topical agent applied is 1 to 1000 mg per dosage.
[15] A method for promoting fibrillin production, the method comprising orally administering the oral administering agent according to [7] or [8].
[16] The method according to [15], wherein an amount of the oral administering agent administered is 1 to 5000 mg per dosage.
[17] A method for promoting elastin fiber production, the method comprising applying the elastin fiber production promoter according to [10] or [11] to a skin or orally administering the elastin fiber production promoter.
[18] The method according to [17], wherein an amount of the elastin fiber production promoter applied is 1 to 1,000 mg, and an amount of the elastin fiber production promoter administered is 1 to 5,000 mg.
[19] A pyrimidine nucleotide or a precursor thereof, for use in promoting fibrillin production.
[20] A pyrimidine nucleotide or a precursor thereof, for use in promoting elastin fiber production.
[21] Use of at least one pyrimidine nucleotide or precursor thereof, for promoting fibrillin production.
[22] Use of at least one pyrimidine nucleotide or precursor thereof, for promoting elastin fiber production.

### [Examples]

While the present invention will be described below with reference to Examples, the present invention is not limited to these Examples in any way.

### (Example 1) Evaluation of Effect of Promoting Fibrillin production

The effect of promoting fibrillin production for pyrimidine nucleotides was evaluated.

The experiment was conducted according to the following procedure.

### (Acquisition of Culture Supernatant of Normal Human Epidermal Cells)

Normal human epidermal cells (NHEK; Kurabo) were seeded in a 96-well plate at a density of 2.5 x 10⁴ cells/well and cultured at 37°C for 24 hours. The medium was replaced with 100 µL of sample-containing medium (HuMedia-KG2; Kurabo) and cultured for 24 hours. After culturing, the culture supernatant was collected. The sample solutions used were 0, 2.5, and 5 (mM) (i.e., 0, 0.0918, and 0.184 (w/v%)) of CMP, 2Na, and 0, 2.5, and 5 (mM) (i.e., 0, 0.092, and 0.184 (w/v%)) of UMP, 2Na.

### (Treatment of Culture Supernatant with Normal Human Fibroblasts)

Normal human fibroblasts (NHDF; Kurabo) were seeded in a 96-well plate at a density of 2.5 x 10⁴ cells/well/100 µL and cultured at 37°C for 24 hours. The cells were then replaced with 100 µL of the culture supernatant obtained in the culture supernatant acquisition step for the three-dimensional cultured epidermis as described above and cultured for 24 hours.

### (Direct Treatment of Normal Human Fibroblasts with Sample)

Normal human fibroblasts (NHDF; Kurabo) were seeded in a 96-well plate at a density of 2.55 x 10⁴ cells/well/100 µL and cultured at 37°C for 24 hours. The medium was then replaced with 100 µL of media (HuMedia-KG2; Kurabo) adjusted to contain 0, 2.5, 5 (mM) (i.e., 0, 0.0918, 0.184 (w/v%)) of CMP, 2Na and 0, 2.5, 5 (mM) (i.e., 0, 0.092, 0.184 (w/v%)) of UMP, 2Na, and cultured for 24 hours.

### (Quantification of Amount of Fibrillin-1)

The culture supernatant from normal human fibroblasts was collected and an amount of fibrillin-1 was quantified by ELISA. The protein amount of the culture supernatant was also measured by BCA protein assay.

The quantified results of the amount of fibrillin-1 when performing the treatment with cytidylic acid are shown in FIG. 1. The quantified results of the amount of fibrillin-1 when performing the treatment with uridylic acid are shown in FIG. 2.

The addition of the culture supernatant from three-dimensionally cultured epidermis treated with the pyrimidine nucleotides promoted the production of fibrillin. The addition of the pyrimidine nucleotides also promoted the production of fibrillin. When comparing the treatment with cytidylic acid with the treatment with uridylic acid, the effect of promoting fibrillin production was comparable.

### (Example 2) Evaluation of mRNA Expression Levels of Collagen Synthesis-related Factors in Fibroblasts Using Culture Supernatant from Three-dimensional Cultured Epidermis

The mRNA expression level of Fibrillin 1 (FBN1), a factor related to elastin synthesis, was quantified by real-time PCR.

### (Acquisition of Culture Supernatant from Three-dimensional Cultured Epidermis)

Three-dimensional cultured epidermis (LabCyte EPI MODEL 24; manufactured by J TEC) was set in a 24-well plate to which 500 µL of the attached assay medium was added, and conditioned overnight at 37°C. 50 µL of sample-containing PBS (-) was applied from the stratum corneum side and cultured for 24 hours. After removing the sample-containing PBS (-), the plate was washed with PBS (-). The medium was replaced with a fresh medium, 50 µL of sample-containing PBS (-) was newly applied from the stratum corneum side, and further cultured for 48 hours. After culturing, the culture supernatant was collected. The concentrations of CMP, 2Na in the respective samples were 0, 1.0, and 2.0 (w/v%), respectively.

### (Treatment of Normal Human Fibroblasts with Culture Supernatant and Quantification of mRNA Expression Level of Fibrillin-1)

Normal human fibroblasts were treated with the culture supernatant by the same method as that of Example 1. RNA was extracted from NHDF using RNeasy Mini Kit (QIAGEN), and mRNA expression analysis for Fibrillin-1 (FBN-1) was performed by real time PCR (ΔΔCt method). Glyceraldehyde 3-phosphate dehydrogenase (GAPDH) was used as an endogenous control.

The results of the analysis of FBN1 mRNA expression are shown in FIG. 3.

It was revealed that the expression level of FBN-1 was increased, depending on the concentration of CMP, 2Na used for the treatment.

### Ex Vivo Evaluation Test Using Freshly Extracted Human Skin

To evaluate the effect on actual skin with greater precision, an ex *vivo* evaluation test was conducted using freshly extracted human skin.

To evaluate the effect on actual human skin with greater precision, an ex *vivo* evaluation test was conducted using freshly extracted human skin.

Abdominal skin tissue was used, which was taken from a 39-year-old Caucasian female patient during cosmetic surgery, after obtaining informed consent for use in research. The extracted skin was placed in a culture plate supplemented with 1.3 ml of D-MEM/Ham's F-12 medium, and conditioned overnight at 37°C in 5% CO₂. An appropriate amount of PBS(-) containing a given concentration of each sample was applied from the stratum corneum side, and further cultured for 24 hours. The treatment was continued for a total of 72 hours while replacing the sample and medium with fresh ones every 24 hours. The concentrations of CMP, 2Na in the respective samples were 0, 1.0, and 2.0 (w/v%) .

After the cultured skin tissue was fixed according to a standard method, skin tissue sections were prepared, elastin fibers were stained with elastica-van Gieson stain, and observed under a microscope.

The results of tissue analysis by elastica-van Gieson staining are shown in FIG. 4.

In the fresh human extracted skin, the cytidylic acid treatment also tended to result in denser elastin fibers.

The results of this Example strongly suggest that fibrillin production is promoted and elastin fiber production is promoted by pyrimidine nucleotides or precursors thereof in actual skin.

## Claims

1. A fibrillin production promoter comprising at least one pyrimidine nucleotide or precursor thereof as an active ingredient.

2. The fibrillin production promoter according to claim 1, wherein a concentration of the pyrimidine nucleotide or precursor thereof is 0.05 to 10.0 (w/v%).

3. The fibrillin production promoter according to claim 1 or 2, wherein the fibrillin production promoter has one or more effects selected from improvement of tactile elasticity of a skin, treatment of emphysema, prevention of progression of emphysema, treatment of arteriosclerosis, and prevention of progression of arteriosclerosis.

4. A cosmetic composition or skin topical agent for promoting fibrillin production, the cosmetic composition or skin topical agent comprising at least one pyrimidine nucleotide or precursor thereof as an active ingredient.

5. The cosmetic composition or skin topical agent according to claim 4, wherein a concentration of the pyrimidine nucleotide or precursor thereof is 0.05 to 10.0 (w/v%) .

6. The cosmetic composition or skin topical agent according to claim 4 or 5, wherein the cosmetic composition or skin topical agent has one or more effects selected from improvement of tactile elasticity of a skin, treatment of emphysema, prevention of progression of emphysema, treatment of arteriosclerosis, and prevention of progression of arteriosclerosis.

7. An oral administering agent for promoting fibrillin production, the oral administering agent comprising at least one pyrimidine nucleotide or precursor thereof as an active ingredient.

8. The oral administering agent according to claim 7, wherein a concentration of the pyrimidine nucleotide or precursor thereof is 0.05 to 10.0 (w/v%).

9. The oral administering agent according to claim 7 or 8, wherein the oral administering agent has one or more effects selected from improvement of tactile elasticity of a skin, treatment of emphysema, prevention of progression of emphysema, treatment of arteriosclerosis, and prevention of progression of arteriosclerosis.

10. An elastin fiber production promoter comprising at least one pyrimidine nucleotide or precursor thereof as an active ingredient.

11. The elastin fiber production promoter according to claim 10, wherein a concentration of the pyrimidine nucleotide or precursor thereof is 0.05 to 10.0 (w/v%).

12. The elastin fiber production promoter according to claim 10 or 11, wherein the elastin fiber production promoter has one or more effects selected from improvement of tactile elasticity of a skin, treatment of emphysema, prevention of progression of emphysema, treatment of arteriosclerosis, and prevention of progression of arteriosclerosis.

13. A method for promoting fibrillin production in a skin, the method comprising applying the cosmetic composition or skin topical agent according to claim 4 or 5 to the skin.

14. The method according to claim 13, wherein an amount of the cosmetic composition or skin topical agent applied is 1 to 1000 mg per dosage.

15. A method for promoting fibrillin production, the method comprising orally administering the oral administering agent according to claim 7 or 8.

16. The method according to claim 15, wherein an amount of the oral administering agent administered is 1 to 5000 mg per dosage.

17. A method for promoting elastin fiber production, the method comprising applying the elastin fiber production promoter according to claim 10 or 11 to a skin or orally administering the elastin fiber production promoter.

18. The method according to claim 17, wherein an amount of the elastin fiber production promoter applied is 1 to 1000 mg, and an amount of the elastin fiber production promoter administered is 1 to 5000 mg.

19. A pyrimidine nucleotide or a precursor thereof, for use in promoting fibrillin production.

20. A pyrimidine nucleotide or a precursor thereof, for use in promoting elastin fiber production.

21. Use of at least one pyrimidine nucleotide or precursor thereof, for promoting fibrillin production.

22. Use of at least one pyrimidine nucleotide or precursor thereof, for promoting elastin fiber production.
